# EUROPEAN PATENT SPECIFICATION

(11) **EP 1 287 164 B1**
(45) Date of publication and mention of the grant of the patent: **13.10.2004**
(21) Application number: 01934121.3
(22) Date of filing: 14.05.2001
(51) Int. Cl.: C12Q 1/68

(54) **A FILTER ELEMENT FOR USE IN SEPARATION OF BIOMATERIALS FROM SOLID CONTAMINANTS**
EIN FILTER ELEMENT ZUR TRENNUNG VON BIOMATERIAL VON FESTEN VERUNREINIGUNGEN
DISPOSITIF DE SEPARATION

(30) Priority: 13.05.2000 GB 0011443
(43) Date of publication of application: 05.03.2003
(73) Proprietor: DNA Research Innovations Limited, Sittingbourne, Kent ME9 8PX (GB)
(72) Inventor: BAKER, Matthew, John, Maidstone, Kent ME15 9UJ (GB)
(74) Representative: Kiddle, Simon John
(86) International application number: PCT/GB2001/002122
(87) International publication number: WO 2001/088185

(56) References cited:
- WO-A-00/09680
- WO-A-00/25922
- WO-A-96/08500
- WO-A-96/41810
- WO-A-97/26540
- US-A- 5 824 224

## Description

### Field of the Invention

The present invention relates to separation devices and in particular to filter elements, devices and methods for separating solid contaminants from a liquid sample containing nucleic acid.

### Background of the Invention

When cells are lysed a crude mixture of soluble and insoluble materials is obtained which often needs to be purified for further analysis or purification. It is often necessary to separate nucleic acid present in the resulting mixture from solid contaminants and this presents a difficult problem in the art, especially where high throughput or automated sample processing are required. In general, the preferred prior art method for separating solid contaminants from nucleic acid containing liquid samples is to use centrifugation to spin down the solid contaminants, leaving a liquid sample containing the nucleic acid. However, while this is an effective technique, it is a slow, labour intensive batch process which is not readily amenable to automation and also requires expensive equipment. Attempts to solve this problem using conventional filters or membranes have been unsuccessful as they need to be supported (especially when wetted) and suffer from clogging, a lack of robustness which adversely affects performance and working life for this type of purification.

WO 97/26540 discloses conical depth filters having a planar filtration surface which are designed to seal within filtration tips.

WO 00/09680 discloses a method of purifying non chromosomal nucleic acid using a conventional depth filter with a planar filtration surface.

### Summary of the Invention

Broadly, the present invention relates to filter elements which can be incorporated in apparatus and uses to separate nucleic acid in liquid samples from solid contaminants. In particular, the present invention relates to filter elements formed from porous materials, especially plastic material with rigid, porous structures that can be formed in shapes other than the conventional disk shaped filters. In preferred embodiments, the filter elements of the present invention are formed with an end wall against which solid contaminants tend to collect, with filtration continuing to take place through one or more unblocked side walls, e.g. in a lateral direction as compared to the flow of the liquid sample against the end wall. In particular, the working life of the filter elements and their adaptability makes the present invention suitable for a range of different situations and can be used in automated systems.

Accordingly, in a first aspect, the present invention provides a filter element formed from a material having a rigid porous structure with a pore size between about 10 and about 200 microns, the filter element having an end wall and one or more side walls extending out of the plane of the end wall, so that when a liquid sample comprising nucleic acid and solid contaminants is introduced into the element, the liquid containing the nucleic acid filters through the side and end walls, while the solid contaminants are retained.

In one embodiment, the filter element is tubular and has a closed end so that when a sample comprising liquid and solid material contacts the filter element, e.g. when it is drawn into a pipette tip or syringe in which the filter is retained, the liquid filters through the side walls and out of the open end of the tube, while the solid material is initially builds up and is retained on the end wall. Thus, in this embodiment, the tubular part of the element forms the side walls, while the outside of closed end of the tube provides the end wall. In use, as solid material builds up in the closed end of the filter element, the liquid containing the nucleic acid can pass through the side walls, allowing filtration to continue and increasing the working life of the filter. In this embodiment, the tube preferably has a uniform circular cross-section. However, other cross-sections will be apparent to those skilled in the art and may be employed to adapt the filter element to fit in apparatus of differing geometries, at locations in the apparatus where a filtration function is required. It would also be possible to include a taper in the filter element, i.e. so that the cross-section varied along its length.

In an alternative embodiment, the filter element is in the form of a plug for spanning an aperture in a piece of apparatus, such as a tube or pipette tip, the plug having an end wall adapted to retain the filter element in the aperture and a side wall protruding from the end wall.
In one preferred embodiment, the plug is approximately T-shaped in cross section, and the side wall protruding from the end wall has a circular cross section. In use, the liquid sample is introduced around the protruding side wall and can filter through the end wall and the protruding side wall. As in the embodiment above, even if the end wall becomes blocked with solid debris, liquid can still pass for some time through the protruding portion as it is raised above the plane of the end wall.

In embodiments where the filter element is adapted to fit inside a pipette tip, a syringe or small tube (e.g. a PCR or centrifuge tube), conveniently, it has a diameter of between about 5 and 15mm and a length of between about 10 and 20 mm. In the first type of tube filter element, preferably the inner diameter of the tube is between about 3 and 5 mm. In the second type of filter element having a protruding side wall, preferably this has a diameter of between about 3 and 6 mm.

In the present invention, the end wall and side walls are defined in relation to the flow of the liquid sample through the apparatus containing the filter element. In preferred embodiments, the side wall(s) of the filter element away from the plane of the end wall so that even if the end wall becomes blocked by layers of solid contaminants or debris building up on it, filtration through the parts of the side wall above the level of the blocking debris is possible.

The filter elements of the present invention therefore provide a solution to the unsolved problem in the prior art of filtering solid debris from liquid samples containing nucleic acid. In preferred configurations, the filter elements are capable of quickly filtering even large volume samples. In comparison to conventional cellulose or glass fibre paper type filters, the filter elements of the invention typically retain less of the liquid sample in the filter, an important advantage in this context as nucleic acid containing liquid sample are often low volume.

The present invention can further be readily adapted for automatic processing in an 8 x 12 format and a standardised pitch where an increased diameter will prevent or hinder multi-channel filtration. The geometry of the filter elements of the invention, which are generally longer than they are wide, works well in these situations, especially when assisted by suction. In this case, typically the length of the filter elements is greater than the width and more preferably at least 1.5 times the width, and still more preferably 2 times the width. The width of the filter element is measured parallel to the plane of the end wall, with the length of the filter element measured parallel to the plane of the side wall(s).

A preferred material for making the filter elements is a porous plastic material such as polypropylene, high density polyethylene (HDPE), polytetrafluoroethane (PTFE), nylon or polyether sulphone. These materials are readily available as sintered plastics and can be formed into the rigid filter elements having the shapes described above. Alternatively, sintered glass could be employed, or an alternative silica, glass or ceramic material.

Preferably, the filter element has a pore size between about 0.01 microns and about 500 microns, more preferably between about 10 microns and about 200 microns, and more preferably between about 20 and about 50 microns. For the filtration of nucleic acid samples, the present inventors have found a pore size between about 10 and about 30 microns to be optimal.

In a further aspect, the present invention provides an apparatus comprising a filter element as described herein.

In a further aspect, the present invention provides a kit comprising a plurality of the filter elements and optionally apparatus into which the filter elements are adapted to fit.

In a further aspect, the present invention provides the use of a filter element as described herein for filtering solid contaminants from a liquid sample containing nucleic acid.

In a further aspect, the present invention provides a method of filtering a liquid sample comprising nucleic acid and one or more solid contaminants, the method comprising passing the sample through a filter element as described herein so that the liquid containing the nucleic acid passes through the filter element and the solid contaminants are retained by the filter element.

In a preferred embodiment, the method includes the initial step of lysing a cell culture to provide a sample and precipitating proteins present in the sample, e.g. with sodium doceyl sulphate (SDS). This commonly used method to prepare samples results in a large amount of solid material that cannot be filtered efficiently using prior art techniques.

According to the invention there is provided a filter element which comprises a sintered material adapted to be moulded to produce a rigid porous structure and the invention also provides a filter which incorporates such a filter element.

Preferably the filter element provides a large surface area, e.g. it is in the form of a hollow plug with the length longer than the width for example with the ratio of length to width of at least 1.5:1, and more preferably at least 2.0:1.0.

An example of a separation device that incorporates the element of the invention uses the element in a multi-channel array, e.g. an 8 x 12 array.

Preferably the device is comprised of a rigid, mouldable, self-supporting porous plug, composed of sintered porous plastic or glass, that can be attached to a pumping or sucking system. The porous plug may be modified chemically or by adsorption of ligands to specifically capture target compounds or remove unwanted materials.

The device may be any shape with a cross sectional area to maximise surface area. Preferably, the devices are longer than they are wide to maximise surface area but maintaining a low diameter for insertion into tubes. The rigid wicks or hollow plugs may be nested inside each other to create a sequence of filters or the hollow plug may contain further particles or microfibers to filter out fine material. Alternatively, a large number of smaller plugs may be used in parallel to provide even larger surface areas.

The device may be combined with chromatographic or affinity purification using standard solid-phases, e.g. ion-exchange, Protein A, antibodies, Streptavidin, etc.

The device is particularly useful for the filtration or purification of biomolecules and cells and especially for separating nucleic acids from liquid mixtures.

In use the liquid to be purified, or from which solid material is to be separated, is drawn up through the filter element into a reservoir or other receptacle.

The invention is particularly useful to remove cell debris from lysed cells.

One embodiment of the invention allows a crude extract of insoluble or soluble materials to be sucked up into a reservoir from a range of laboratory test tubes such as PCR tubes, micro-titre plates, centrifuge tubes and any standard container from a few microlitres to litre volumes. Once the fluids have been drawn up through the device then further processing or purification is possible.

The shape and design of the device is flexible and may be formed by moulding the porous material into any shape or structure.

It is a feature of the device that it can maximise flow rates, prevent clogging or blockages and presents a larger than normal surface area parallel to the fluid in both directions while maintaining a narrow diameter for multi-channel fluid handling systems.

The filter element is self supporting and rigid, not requiring other supporting casing or moulds for it to work. Therefore it can be placed over the outside of a dispensing or aspirating system and removed to discard the filter element or to process the material captured by the filter element.

The filter element can be incorporated internally in a pipette or can be attached to the end of a pipette so that liquid can be sucked up through the filter element into the pipette.

Several designs of plug have been tested for efficient separation of contaminants, using cellulose or glass microfiber membranes. Another variation for microbial purifications is that the cells or debris can be concentrated or removed by using specific ligands such as antibodies, polymixins, lectins, enzymes, boronic acid or other affinity materials.

The device is especially suitable for biological samples from medical research to food and agriculture where insoluble materials need to be removed before purification of the target analyte.

Embodiments of the present invention will now be described in more detail by way of example and not limitation with reference to the accompanying drawings.

### Brief Description of the Drawings

Figures 1 and 2 shows embodiments of the invention and in place in different apparatus where a filtration function is needed.
Figures 3a and 3b show an example of a closed tubular embodiment of the invention.
Figure 4 shows an example of a filter element with a protruding side wall, in place in a centrifuge tube.
Figure 5 shows a perspective view of the filter element of Figure 4.
Figure 6 shows the filter element of Figure 4 or Figure 5 in place in a pipette tip.

### Detailed Description

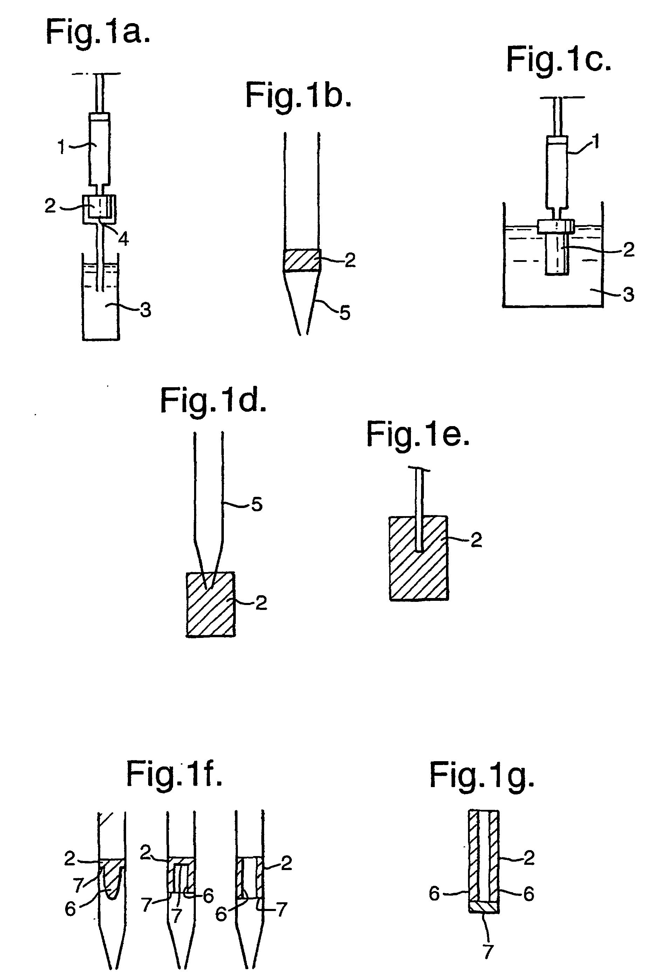

Figure 1a shows a syringe 1 sucking up a plasmid preparation 3 through a hollow porous plastic plug 2 with the bottom end 4 closed. The debris remain on the outside of the plug allowing the DNA to travel through into the syringe barrel. The plug 2 avoids immediate blockage by presenting a large surface area and the external housing of the cartridge allows the material to travel up the plug without forcing the particulate material into the pores. The plug 2 only blocks when the liquid has travelled all the way to the top of the housing. The filtration device may then be removed and the liquid transferred into a new tube. The use of this embodiment is described in Examples 1, 2 and 3.

The above system allows fully automated extraction of plasmid DNA from crude bacterial lysates. The reservoir above the filter holds the clarified fluid for purification on affinity media of come tyre.

The device has many applications whether in a manual or automated operation and even with larger volumes a standard eight by twelve array of tubes can be processed without a pitch change in a multi-channel instrument.

Figure 1b shows a plug 2 in a pipette tip 5 so that, if the plasmid lysate is clarified, then the plug may be modified to capture DNA directly.

Figure 1c shows a modification of the Figure 1a device without the external housing surrounding the hollow plug 2.

Figure 1d shows a porous plug or hollow plug 2 fitted on externally to allow easy removal while maintaining the fluid in the pipette tip 5.

Figure 1e shows a porous plug fitted onto a solid pin or moulding that can be dipped into a tube to capture biomolecules. This could be extended to an 8 x 12 microtitre format or PCR tube array.

Figure 1f shows embodiments of the plugs 2 of the invention which are shaped to increase the surface area in standard pipette tips, with end walls 7 and side walls 6 marked.

Figure 1g shows the outline of a hollow porous plug made from sintered plastic or glass, showing the side walls 6 and end wall 7 of the plug 2. The device is rigid enough to support itself and the open end is fitted onto the sucking and pumping system. This design maximises surface area vertically and reduces the pitch between adjacent devices, e.g. in a multi-channel system.

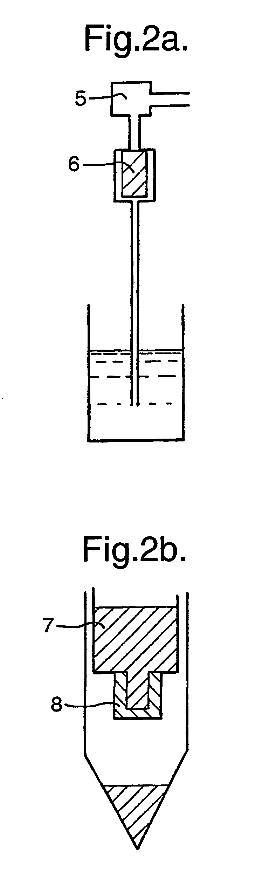

In Figure 2a there is a pump 5 that can generate continuous liquid flow through the device incorporating plug 6 so that the liquid may be re-circulated if required.

Figure 2b shows how the device may be used with centrifugation tubes to increase the surface area compared to a flat disc where 7 is the liquid and 8 is the filter element.

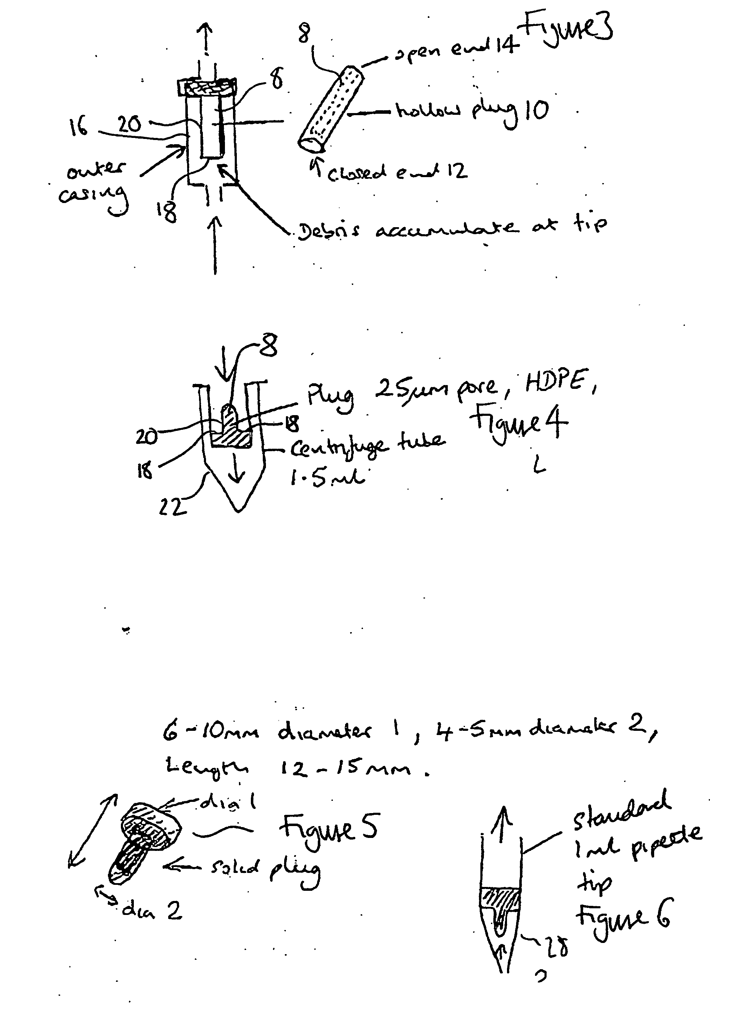

Figure 3 shows an embodiment of the invention which uses a filter element 8 having the form of a tubular plug 10 having a closed end 12 and an open end 14, with arrows showing the flow of a liquid sample through the filter element. The external surface of the closed end 12 provides an end wall 18 and the curved surface of the tubular part of the plug defines a side wall 20. The filter element 8 is retained in a syringe, pipette or other tube 16 with the closed end 12 of the plug directed towards the flow of the sample into the tube 16. When a sample encounters the filter element 8, solid contaminants, such as cell debris, will tend to be retained on the end wall 18, while liquid containing nucleic acid and other soluble components of the sample can pass through the side wall 20 into the hollow core of the tube and out of the open end 14 for further purification or analysis, the hollow core helping the efficiency of filtration by reducing the transmembrane pressure experienced by the sample across the filter element. The tendency of the end wall of the device to capture debris and the high surface area that results from using a porous plastic material to form the filter element 8 means that the rapid clogging observed with prior art filtration techniques is avoided, and that instead layers of solid debris tend to build up on the end wall of the device.

Figure 4 shows an alternative form of filter element 8, in this case designed to fit across the opening of a centrifuge or PCR tube 22. The filter element has an end wall 18 which spans the opening 24 of the tube 22 and a generally cylindrical central portion 26 having a side wall 20 which protrudes towards the direction of sample flow. In use, a sample introduced into the open space at the top of the tube 22 can filter through the end wall 18 and side wall 20, with debris again tending over time to collect against the end wall, leaving the liquid free to filter through the side wall 20 as the protruding central portion stands clear of the build up the solid.debris.
Figure 5 shows a perspective view of the filter element 8, while Figure 6 shows the filter element in place in a pipette tip 28. As in Figure 3, the arrows indicate the direction of liquid flow through the filter element.

### Example 1

### Extraction of nucleic acid from bacterial lysates

This example demonstrates the filtration of bacterial lysates and the purification of plasmid DNA. An overnight culture of *E. Coli* possessing a plasmid was lysed using a modified alkaline lysis method and the cell debris were removed by sucking the fluid up through a rigid 20 micron porous sintered plastic plug using embodiment shown in Figure 1a. The debris was retained by the filter allowing the plasmid DNA to travel into the reservoir in this case a syringe barrel or pipette tip. The plasmid DNA was captured on the modified plug and washed free of contaminants with water before recovery in a small volume of Tris.HCl pH8.5. The plug was removed and the fluid allowed to be pumped down through another plug covalently modified with polyhistidine according to patent application WO 99/29703 (DNA Research Instruments Ltd).

### Example 2

### Extraction of nucleic acid from natural source material

2 grams of cabbage leaves were homogenised in warm sodium dodecyl sulphate (SDS) to release the nucleic acids. Following potassium acetate/potassium chloride precipitation, the fluid was sucked up a twenty micron plug to remove the insoluble material and the DNA extracted using a polyhistidine affinity membrane combined in the device.

### Example 3

### Extraction of nucleic acid from white blood cells.

Affinity capture of analytes such as nucleic acids, proteins, cells, organelles and other compounds were performed using this device. The capture or removal of white blood cells from whole anti-coagulated blood can be performed by mixing the blood with ammonium bicarbonate buffers containing high levels of non ionic detergents such as 1% (v/v) Tween 20.

The blood is sucked through a hollow plug allowing the cells to bind and the contaminants washed off using the same buffer. The cells may then be processed for collection of DNA, RNA or analysed by a known method.
This system can be used in combination with collection of blood samples directly from the donor either using a needle and syringe or a vacuum tube to suck the blood through the porous material. The porous material may be used to store the captured substance or transferred to another storage tube without having to release the captured substance.

### Example 4

### Extraction of plasmid DNA from culture

An overnight culture of E.Coli/PUC19 was prepared and 25ml centrifuged to pellet the cells. The cell pellet was resuspended in 2ml of 10mM Tris HCl containing Rnase A and mixed with a further 2ml of 0.2M NaOH with 1%SDS to lyse the cells and release the plasmid DNA. The cellular debris and SDS was then precipitated with 2ml of 3M potassium Acetate pH4 and left to stand for 5 minutes.
The liquid was separated from the precipitate by a filter element of the type depicted in Figure 3, using a 25 micron pore plastic hollow plug inside a 3ml cartridge about 4cm long and 1cm in diameter. The cartridge tip was dipped into the mixture and the liquid sucked up through the filter into a syringe barrel. The precipitate remained on the outside of the porous plug producing a clear liquid in the syringe barrel in about 1 minute. The total yield of liquid was 5.5ml, over 90% recovery from the starting material. The filtered liquid was then processed to obtain pure plasmid using magnetic beads derivatised with Bis-Tris or by alcohol precipitation.

The filter plug was then regenerated by pumping water back through until all the precipitated was washed away.
This can then be used for repeat experiments or continuous flow operation.

The same experiment was repeated except the cartridge was inverted and the precipitated mixture was pumped from the syringe barrel down through the plug. The precipitate collected at the base of the plug leaving the majority of the filter unclogged to allow easy flow of liquid. In this case, recovery of liquid was even better at about 95% yield.

The device was used as a pre-filter on the same volume of plasmid preparation to allow filtering down to 1 micron or 0.45 micron. By incorporating an additional filter after the plug, the device allowed filtration to 1 micron or less with 80% recovery of fluid and a 5 minute filtration time.

### Example 5

### Comparison with conventional filtration

Instead of using the.filter element described above, a standard 25 micron pore frit made of porous plastic sheet with a diameter of 25mm was inserted into a 30ml syringe barrel with spacing collars to hold it in place and expose the surface to the liquid.

The precipitated mixture from the plasmid preparation was either sucked up through the frit or pushed through. In both cases, only 50% of the fluid was recovered due to almost immediate clogging of the membrane. In an attempt to prevent clogging, stacks of filter paper were placed in front of the 25mm frit, but the performance in terms of yield of liquid and flow rates could not be improved.

Thus, if standard 25mm glass fibre or paper pre-filters are used, clogging occurs very quickly and recovery of liquid is slow. In many cases, this means that it is impractical to use filtration to remove solid contaminants from liquid samples containing nucleic acid.

### Filtration in microtubes using centrifugation vacuum manifolds

A conventional frit or filter from a 1.5ml centrifuge filter tube was replaced with a porous 25 micron plug inverted to increase the surface area and prevent clogging. A 5ml culture was precipitated as described above reducing the original volume to about 1ml ready for filtration. The mixture was tipped into the tube with the plug and either placed on a vacuum manifold or centrifuged for 3 minutes. The fluid was easily collected with no signs of clogging and 90% of liquid was recovered.

With the original filter material in place, clogging with this sample volume occurred immediately and only about 50% of the original was recovered.

### Filtration using pipette tips

A standard 1ml pipette tip was used to filter a 5ml plasmid preparation by inserting a 25 micron plug into the tip. The mixture could either be sucked up or pumped through within 1 minute with 80% recovery of liquid.
This was then repeated using a multi-channel pipettor for filtering 8 samples simultaneously.

## Claims

1. A filter element formed from a material having a rigid porous structure with a pore size between about 10 and about 200 microns, the filter element having an end wall and one or more side walls extending out of the plane of the end wall, so that when a liquid sample comprising nucleic acid and solid contaminants is introduced into the element, the liquid containing the nucleic acid filters through the side and end walls, while the solid contaminants are retained.

2. The filter element of claim 1, wherein the filter element is a close ended tube with the side wall defined by a curved wall of the tube and end wall defined by the outside of closed end of the tube.

3. The filter element of claim 1, wherein the filter element is in the form of a plug for spanning an aperture in a piece of apparatus, and having an end wall from which a side wall protrudes.

4. The filter element of any one of claims 1 to 3 which is adapted to fit into a pipette tip, a syringe or a PCR or centrifuge tube.

5. The filter element of any one of the preceding claims, wherein the material is a plastic.

6. The filter element of any one of the preceding claims wherein the plastic is polypropylene, high density polyethylene (HDPE), polytetrafluoroethane (PTFE), nylon or polyether sulphone.

7. The filter element of claim 5 or claim 6, wherein the plastic is a sintered plastic.

8. The filter element of any one of the preceding claims, wherein the pore size is between about 20 and about 50 microns.

9. The filter element of any one of the preceding claims, wherein the length of filter element is greater than its width.

10. The filter element of claim 9, wherein the ratio of length to width is at least 1.5:1.

11. Apparatus comprising a filter element of any one of the preceding claims.

12. The apparatus of claim 11 which is a pipette tip, a multipipettor, a syringe, or a PCR or centrifugation tube.

13. A kit comprising a plurality of the filter elements of any one of claims 1 to 10 and optionally apparatus into which the filter elements are adapted to fit.

14. Use of a filter element of any one of claims 1 to 10 for filtering solid contaminants from a liquid sample containing nucleic acid.

15. A method of filtering a liquid sample comprising nucleic acid and one or more solid contaminants, the method comprising passing the sample through a filter element of any one of claims 1 to 10 so that the liquid containing the nucleic acid passes through the filter element and the solid contaminants are retained by the filter element.

16. The method of claim 15, wherein the liquid sample is a cell culture and the method includes the initial step of lysing a cell culture and precipitating proteins present in the sample.

17. The method of claim 15 or claim 16, comprising the step of sucking the liquid sample through the filter element.

## Patentansprüche

1. Filterelement, das aus einem Material mit harter poröser Struktur mit einer Porengröße zwischen etwa 10 und etwa 200 Mikrometern hergestellt ist, wobei das Filterelement eine Endwand und eine oder mehrere Seitenwände besitzt, die sich aus der Ebene der Endwand heraus erstrecken, sodass wenn eine flüssige Probe, die Nucleinsäure und feste Verunreinigungen umfasst, in das Element eingebracht wird, die Nucleinsäure enthaltende Flüssigkeit durch die Seiten- und Endwände hindurchtritt, während die festen Verunreinigungen festgehalten werden.

2. Filterelement nach Anspruch 1, worin das Filterelement ein Rohr mit geschlossenem Ende ist, dessen Seitenwand durch eine gekrümmte Wand des Rohrs definiert ist und dessen Endwand durch die Außenseite des geschlossenen Endes des Rohrs definiert ist.

3. Filterelement nach Anspruch 1, worin das Filterelement die Form eines Stöpsels zum Verschließen einer Öffnung in einem Teil einer Vorrichtung aufweist und eine Endwand aufweist, von der aus eine Seitenwand vorsteht.

4. Filterelement nach einem der Ansprüche 1 bis 3, das angepasst ist, um in die Spitze einer Pipette, in eine Spritze, ein PCR-Röhrchen oder ein Zentrifugenglas hineinzupassen.

5. Filterelement nach einem der vorangegangenen Ansprüche, worin das Material ein Kunststoff ist.

6. Filterelement nach einem der vorangegangenen Ansprüche, worin der Kunststoff Polypropylen, Polyethylen von hoher Dichte (HDPE), Polytetrafluorethan (PTFE), Nylon oder Polyethersulfon ist.

7. Filterelement nach Anspruch 5 oder 6, worin der Kunststoff ein Sinterkunststoff ist.

8. Filterelement nach einem der vorangegangenen Ansprüche, worin die Porengröße zwischen etwa 20 und etwa 50 Mikrometer beträgt.

9. Filterelement nach einem der vorangegangenen Ansprüche, worin die Länge des Filterelements größer als seine Breite ist.

10. Filterelement nach Anspruch 9, worin das Verhältnis der Länge zur Breite zumindest 1,5:1 beträgt.

11. Vorrichtung, umfassend ein Filterelement nach einem der vorangegangenen Ansprüche.

12. Vorrichtung nach Anspruch 11, welches eine Pipettenspitze, eine Multipipette, eine Spritze, ein PCR-Röhrchen oder ein Zentrifugenglas ist.

13. Bausatz, umfassend eine Vielzahl der Filterelemente nach einem der Ansprüche 1 bis 10 und gegebenenfalls eine Vorrichtung, in dem die hierfür angepassten Filterelemente hineinpassen.

14. Verwendung eines Filterelements nach einem der Ansprüche 1 bis 10 zum Abfiltrieren von festen Verunreinigungen aus einer flüssigen Probe, die Nucleinsäure enthält.

15. Verfahren zum Filtrieren einer flüssigen Probe, die Nucleinsäure und eine oder mehrere feste Verunreinigungen enthält, wobei das Verfahren das Hindurchtretenlassen der Probe durch ein Filterelement nach einem der Ansprüche 1 bis 10 umfasst, sodass die Nucleinsäure-hältige Flüssigkeit durch das Filterelement durchtritt und die festen Verunreinigungen vom Filterelement zurückgehalten werden.

16. Verfahren nach Anspruch 15, worin die flüssige Probe eine Zellkultur ist und das Verfahren den Anfangs-Schritt des Lysierens einer Zellkultur und des Ausfällens von Proteinen, die in der Probe gegenwärtig sind, umfasst.

17. Verfahren nach Anspruch 15 oder Anspruch 16, umfassend den Schritt des Saugens der flüssigen Probe durch das Filterelement hindurch.

## Revendications

1. Elément de filtration réalisé en un matériau ayant une structure poreuse rigide avec une taille de pore entre environ 10 et environ 200 microns, l'élément de filtration ayant une paroi d'extrémité et une ou plusieurs parois latérales s'étendant hors du plan de la paroi d'extrémité de sorte que lorsqu'un échantillon liquide comprenant de l'acide nucléique et des contaminants solides est introduit dans l'élément, le liquide contenant l'acide nucléique est filtré à travers les parois latérales et d'extrémité pendant que les contaminants solides sont retenus.

2. Elément de filtration selon la revendication 1, où l'élément de filtration est un tube à extrémité fermée avec la paroi latérale définie par une paroi courbée du tube et une paroi d' extrémité définie par l'extérieur de l'extrémité fermée du tube.

3. Elément de filtration selon la revendication 1, où l'élément de filtration se présente sous la forme d'un bouchon pour recouvrir une ouverture dans une pièce d'appareil, et possédant une paroi d'extrémité à partir de laquelle une paroi latérale fait saillie.

4. Elément de filtration selon l'une des revendications 1 à 3, qui est conçu pour s'adapter dans une pointe de pipette, une seringue ou un PCR ou tube centrifuge.

5. Elément de filtration selon l'une des revendications précédentes, où le matériau est un plastique.

6. Elément de filtration selon l'une des revendications précédentes, où le plastique est du polypropylène, du polyéthylène haute densité (HDPE), du polytétrafluoroéthane (PTFE), du nylon ou du sulfone de polyéther.

7. Elément de filtration selon la revendication 5 ou la revendication 6, où le plastique est un plastique fritté.

8. Elément de filtration selon l'une des revendications précédentes, où la taille des pores est entre environ 20 et environ 50 microns.

9. Elément de filtration selon l'une des revendications précédentes, où la longueur de l'élément de filtration est plus grande que sa largeur.

10. Elément de filtration selon la revendication 9, où le rapport de la longueur à la largeur est au moins de 1,5:1.

11. Appareil comprenant un élément de filtration selon l'une des revendications précédentes.

12. Appareil selon la revendication 11, qui est une pointe de pipette, une multi-pipette, une seringue ou un PCR ou un tube de centrifugation.

13. Kit comprenant plusieurs éléments de filtration selon l'une des revendications 1 à 10 et en option un appareil dans lequel les éléments de filtration sont aptes à être insérés.

14. Utilisation d'un élément de filtration selon l'une des revendications 1 à 10 pour filtrer des contaminants solides d'un échantillon liquide contenant de l'acide nucléique.

15. Procédé de filtration d'un échantillon liquide comprenant de l'acide nucléique et un ou plusieurs contaminants solides, le procédé comprenant le passage de l'échantillon à travers un élément de filtration selon l'une des revendications 1 à 10, de telle sorte que le liquide contenant l'acide nucléique passe à travers l'élément de filtration, et que les contaminants solides sont retenus par l'élément de filtration.

16. Procédé selon la revendication 15, où l'échantillon liquide est une culture de cellules, et le procédé comprend l'étape initiale consistant à lyser une culture de cellules et à précipiter les protéines présentes dans l'échantillon.

17. Procédé selon la revendication 15 ou la revendication 16, comprenant l'étape consistant à aspirer l'échantillon liquide à travers l'élément de filtration.
